# EUROPEAN PATENT APPLICATION

(11) **EP 4 822 356 A1**
(43) Date of publication of application: **14.10.2026**
(21) Application number: 25818707.9
(22) Date of filing: 21.01.2025
(51) Int. Cl.: G01N 35/00, G01N 21/76

(54) **FULLY-AUTOMATIC CHEMILUMINESCENCE APPARATUS FOR MULTI-CHANNEL MULTIPLEXED POCT**

(30) Priority: 06.06.2024 CN 202410732948
(71) Applicant: Drophil Biotech (Shenzhen) Company Limited, Shenzhen, Guangdong 518122 (CN)
(72) Inventor: WANG, Dong, Shenzhen, Guangdong 518122 (CN); ZHANG, Mingxing, Shenzhen, Guangdong 518122 (CN); LIN, Qingliang, Shenzhen, Guangdong 518122 (CN); MA, Yuanbin, Shenzhen, Guangdong 518122 (CN)
(74) Representative: Metida
(86) International application number: PCT/CN2025/073504
(87) International publication number: WO 2025/251642

(57) **Abstract**

A multi-channel multi-assay POCT fully automatic chemiluminescence device includes: a mounting frame; a reaction chamber assembly for loading a multi-assay reagent strip, which is configured to detect at least two markers, and the multi-assay reagent strip includes a liquid sealing region, which includes a common liquid region and a dedicated liquid region; a multi-channel multi-assay parallel pre-processing apparatus, including at least two rows of magnetic rods, where each row of magnetic rods includes at least two magnetic rods; a film breaking assembly, configured to break a sealing film of the common liquid region; and a PMT assembly, where the reaction chamber assembly moves the multi-assay reagent strip to the PMT assembly, so as to detect a luminescence value of a detection position on the multi-assay reagent strip. In this application, multi-channel parallel processing is implemented while multiple assays are implemented.

## Description

### TECHNICAL FIELD

This application relates to the field of medical device technologies, and in particular, to a multi-channel multi-assay POCT fully automatic chemiluminescence device.

### BACKGROUND

Chemiluminescence immunoassay technology, as a rapidly developing non-radioactive immunoassay technology, has become one of the important directions of immunological detection. The principle of chemiluminescence immunoassay is to directly measure immune reactions by using chemiluminescence signals. Chemiluminescence devices applying the chemiluminescence immunoassay technology have advantages such as high sensitivity, high repeatability, high accuracy, high specificity, and a wide linearity range.

Point-Of-Care Testing (POCT) is a testing method that performs clinical detection immediately at a specimen collection site, eliminates complex processing procedures when specimens are tested in a laboratory, and thus quickly obtains test results. It features instrument programming, simple operation, and fast result reporting.

Currently, a multi-channel multi-assay POCT fully automatic chemiluminescence device is manufactured by applying the chemiluminescence immunoassay technology to POCT products. Such a device has advantages of instant detection and rapid acquisition of diagnostic results, and is rapidly and widely promoted in the in vitro diagnostic industry.

The multi-channel multi-assay POCT fully automatic chemiluminescence device generally includes a reaction chamber module and a multi-channel parallel pre-processing module. The reaction chamber module is loaded with reagent strips sealed with various reagents, and moves the reagent strips to the multi-channel parallel pre-processing module for corresponding processing, such as breaking a sealing film on a reagent well and transferring magnetic particles. A length of the reagent strip depends on the number of detection items. As the number of the detection items increases, the reagents sealed in the reagent strip also need to increase accordingly, which results in an increase in the length of the reagent strip.

When the device is used for detection and analysis of a plurality of items, a distance that the reaction chamber module needs to move with the reagent strip in the device increases, so that the reagent strip can be moved to the multi-channel parallel pre-processing module for corresponding processing. For example, a long movement distance is required to enable the multi-channel parallel pre-processing apparatus to break the sealing film on the reagent strip. This will lead to an increase in a volume of the device, which is not conducive to miniaturization of the device, and will also increase the time for detection and the acquisition of the diagnostic results.

In addition, the current multi-channel parallel pre-processing module can only transfer the magnetic particles in one well position on the reagent strip at a time. If the magnetic particles in a plurality of well positions need to be transferred, a plurality of operations are required, which increases a risk of cross-contamination and increases the transfer time of the magnetic particles, and due to the long time taken for a plurality of transfers of the magnetic particles, the analysis efficiency of the device is affected.

### TECHNICAL PROBLEMS

One of objectives of embodiments of this application is to provide a multi-channel multi-assay POCT fully automatic chemiluminescence device, aiming to solve problems about how to control a device volume and ensure a device detection speed when a reagent strip is lengthened to meet a plurality of detection requirements, as well as problems that an existing multi-channel parallel pre-processing module requires a plurality of operations when transferring magnetic particles in a plurality of well positions, which increases the risk of cross-contamination and transfer time of the magnetic particles, and affects analysis efficiency of the device.

### TECHNICAL SOLUTIONS

Embodiments of this application are implemented as follows: a multi-channel multi-assay POCT fully automatic chemiluminescence device includes:
a mounting frame;
a reaction chamber assembly disposed on the mounting frame and configured to load multi-assay reagent strips, where the reaction chamber assembly is reciprocally movable in a length direction of the mounting frame, the multi-assay reagent strip is configured to detect at least two markers, the multi-assay reagent strip includes a liquid sealing region, and the liquid sealing region includes a common liquid region and a dedicated liquid region that are distributed in a length direction of the multi-assay reagent strip;
a multi-channel multi-assay parallel pre-processing apparatus disposed on the mounting frame, where the multi-channel multi-assay parallel pre-processing apparatus includes at least two rows of magnetic rods arranged side by side in a movement direction of the reaction chamber assembly, each row of magnetic rods includes at least two magnetic rods, and the at least two rows of magnetic rods are configured to transfer magnetic particles in adjacent well positions of the multi-assay reagent strip in the liquid sealing region;
a film breaking assembly disposed on the mounting frame and located at a rear end of the multi-channel multi-assay parallel pre-processing apparatus, where the film breaking assembly is configured to break a sealing film of the common liquid region; and
a PMT assembly disposed on the mounting frame and located at a rear end of the film breaking assembly, where the reaction chamber assembly is configured to move the multi-assay reagent strip to the PMT assembly, and the PMT assembly is configured to detect a luminescence value of a detection position of the multi-assay reagent strip.

Further, the multi-channel multi-assay parallel pre-processing apparatus includes:
a gantry disposed on a bottom plate of the mounting frame, where the top of the gantry is provided with a first driving element;
a specimen extraction mechanism disposed on the gantry, where the specimen extraction mechanism is in transmission connection to the first driving element and may ascend and descend under the drive of the first driving element to aspirate and dispense specimens on the multi-assay reagent strips; and
a specimen pre-processing mechanism disposed on the gantry, where the specimen pre-processing mechanism is in transmission connection to the first driving element and may ascend and descend under the drive of the first driving element, and the at least two rows of magnetic rods are disposed on the specimen pre-processing mechanism.

Further, the specimen pre-processing mechanism includes:
a first mounting frame disposed on the gantry and being in transmission connection to the first driving element, where the first driving element may drive the first mounting frame to ascend and descend;
a second driving element disposed on the first mounting frame; and
a pre-processing assembly disposed on the first mounting frame and being in transmission connection to the second driving element, where the at least two rows of magnetic rods are disposed on the pre-processing assembly and are in transmission connection to the second driving element, and the second driving element may drive the at least two rows of magnetic rods to ascend and descend to transfer the magnetic particles.

Further, the pre-processing assembly includes:
a magnetic rod device disposed on the first mounting frame, where a plurality of movable through holes are formed in the magnetic rod device, a plurality of first loading heads for loading disposable magnetic separation sleeves are disposed at the bottom of the magnetic rod device, and the plurality of first loading heads communicate with the plurality of movable through holes in a one-to-one manner;
a first push plate disposed above the magnetic rod device, where the first push plate is in transmission connection to the second driving element; and
a plurality of connecting rods disposed on the first push plate, where the at least two rows of magnetic rods are disposed at free ends of the plurality of connecting rods in a one-to-one manner, and the second driving element may drive the first push plate to drive the magnetic rods to ascend and descend in the corresponding movable through holes, so as to be spaced apart from or pass through the first loading heads.

Further, the magnetic rods are arranged in a plurality of rows, and the plurality of rows of magnetic rods are evenly spaced in a width direction of the first push plate; and
each row of magnetic rods includes a plurality of magnetic rods, and the plurality of magnetic rods in each row are evenly spaced in a length direction of the first push plate.

Further, the specimen extraction mechanism includes:
a second mounting frame disposed on the gantry and being in transmission connection to the first driving element, where the first driving element may drive the second mounting frame to ascend and descend;
a third driving element disposed on the second mounting frame; and
an extraction assembly disposed on the second mounting frame and being in transmission connection to the third driving element, where the third driving element may drive the extraction assembly to ascend and descend to aspirate and dispense the specimens.

Further, the extraction assembly includes:
an injection device disposed on the second mounting frame, where a plurality of injector cavities are formed in the injection device, a plurality of second loading heads for loading tip heads are disposed at the bottom of the injection device, and the plurality of second loading heads communicate with the plurality of injector cavities in a one-to-one manner;
a second push plate disposed above the injection device, where the second push plate is in transmission connection to the third driving element; and
a plurality of piston rods disposed on the second push plate, where the piston rods are movably and sealingly connected to the injector cavities, and the third driving element may drive the second push plate to drive the piston rods to ascend and descend in the injector cavities to perform aspirating and dispensing actions.

Further, the specimen extraction mechanism also includes:
push rods that penetrate through the injection device in a liftable manner, where top ends of the push rods are spaced apart from the second push plate;
elastic elements sleeving the push rods located in the injection device, where the elastic elements are simultaneously stretched or compressed when the push rods ascend or descend relative to the injection device; and
a separation plate disposed at the bottom of the injection device, where the second loading heads pass through the separation plate, bottom ends of the push rods are connected to the separation plate, and the push rods may drive the separation plate to ascend and descend relative to the second loading heads.

Further, the plurality of injector cavities are evenly spaced in a length direction of the injection device, the plurality of second loading heads are evenly spaced in the length direction of the injection device, and the plurality of piston rods are evenly spaced in a length direction of the second push plate.

Further, the specimen extraction mechanism is opposite to the specimen pre-processing mechanism, the specimen extraction mechanism is located in front of the specimen pre-processing mechanism, and the first driving element, the second driving element, and the third driving element are linearly arranged on the top of the gantry.

Further, the gantry is provided with a movable space, a transmission shaft of the first driving element passes through the movable space, a transmission plate sleeves the transmission shaft, and when the transmission shaft acts, the transmission plate can ascend and descend in the movable space.

A first connecting portion is disposed on one side of the first mounting frame towards the gantry, a second connecting portion is disposed on one side of the second mounting frame towards the gantry, and both the first connecting portion and the second connecting portion are connected to the transmission plate.

Further, a first slide seat is disposed on one side of the first mounting frame towards the gantry, a first slide rail is disposed on one side of the gantry towards the first mounting frame, the first slide rail extends in a height direction of the gantry, and first slide seat is slidably connected to the first slide rail.

A second slide seat is disposed on one side of the second mounting frame towards the gantry, a second slide rail is disposed on one side of the gantry towards the second mounting frame, the second slide rail extends in the height direction of the gantry, and the second slide seat is slidably connected to the second slide rail.

Further, a first sensing member is disposed on one side of the top of the first mounting frame, a first detector is disposed on one side of the top of the gantry, and the first sensing member may generate induction or disconnect induction with the first detector as the first mounting frame ascends and descends.

A second sensing member is disposed on one side of the top of the second mounting frame, a second detector is disposed on one side of the top of the gantry, and the second sensing member may generate induction or disconnect induction with the second detector as the second mounting frame ascends and descends.

Further, the reaction chamber assembly includes:
a guide rail disposed on the bottom plate of the mounting frame;
a reaction chamber body disposed on the guide rail, where a guide block that is slidably connected to the guide rail is disposed at the bottom of the reaction chamber body, a plurality of accommodation grooves evenly spaced and configured to load the multi-assay reagent strips are disposed on the reaction chamber body, and the accommodation grooves extend in a length direction of the reaction chamber body;
a fourth driving element disposed on the bottom plate of the mounting frame and located at a rear end of the guide rail, where a first driving pulley is disposed on a transmission shaft of the fourth driving element;
a mounting seat disposed on the bottom plate of the mounting frame and located at a front end of the guide rail, where a first driven pulley is disposed on the mounting seat, and the first driving pulley is in transmission connection to the first driven pulley through a belt; and
a first synchronous belt pressing plate disposed on one side of the reaction chamber body, where the first synchronous belt pressing plate is connected to the belt.

Further, the PMT assembly includes:
a mounting bracket disposed on the bottom plate of the mounting frame;
a third mounting frame disposed on the mounting bracket;
a fourth mounting frame disposed at the bottom of the third mounting frame;
a PMT module disposed on the fourth mounting frame;
a fifth driving element disposed on the top of the third mounting frame and being in transmission connection to the fourth mounting frame, where the fifth driving element may drive the fourth mounting frame to ascend and descend so as to drive the PMT module to ascend and descend;
a sixth driving element and a second driven pulley respectively disposed on two sides of the top of the mounting bracket, where a transmission shaft of the sixth driving element is provided with a second driving pulley, and the second driving pulley is in transmission connection to the second driven pulley through a belt; and
a second synchronous belt pressing plate disposed on the third mounting frame, where the second synchronous belt pressing plate is connected to the belt.

Further, the film breaking assembly includes:
a seventh driving element disposed on the top of the mounting bracket;
a fifth mounting frame disposed on the mounting bracket and located below the seventh driving element, where the fifth mounting frame is in transmission connection to the seventh driving element and can ascend and descend under the drive of the seventh driving element; and
a plurality of film breaking heads disposed at the bottom of the fifth mounting frame, where the plurality of film breaking heads are evenly spaced in a length direction of the fifth mounting frame.

Further, the multi-assay reagent strip includes:
a reagent strip body, where the liquid sealing region is disposed on the reagent strip body;
a handle disposed at one end of the reagent strip body; and
a specimen well, a tip head, at least two disposable magnetic separation sleeves, the dedicated liquid region, and the common liquid region, which are disposed on the reagent strip body and sequentially arranged from the handle to the other end of the reagent strip body.

The dedicated liquid region includes at least two magnetic particle-labeled ligands and at least two enzyme-labeled ligands, and the common liquid region includes a cleaning solution and a luminescent substrate solution.

In the embodiments of this application, the multi-channel multi-assay parallel pre-processing apparatus includes the at least two rows of magnetic rods for transferring the magnetic particles, and each row of magnetic rods includes the at least two magnetic rods. That is, the multi-channel multi-assay parallel pre-processing apparatus may simultaneously transfer the magnetic particles in at least two adjacent reagent wells on a single multi-assay reagent strip, and transfer the magnetic particles in adjacent reagent wells of at least two multi-assay reagent strips arranged in parallel. In this way, the at least two markers can be analyzed and detected, and multiple assays can be implemented while achieving multi-channel parallel processing. This can reduce the risk of cross-contamination and shorten the time for transferring the magnetic particles, thereby ensuring the analysis efficiency of the device.

Further, based on a structure of the device and combined with a dedicated design of the multi-assay reagent strip, an operation of breaking a sealing film of the multi-assay reagent strip does not need to be completely performed by the multi-channel multi-assay parallel pre-processing apparatus. Instead, the sealing film of the common liquid region may be broken by the film breaking assembly at the rear end. The reaction chamber assembly can implement targeted film breaking without moving the multi-assay reagent strip for a long distance. This reduces a repeated and long-distance movement of the reaction chamber assembly, and reduces repeated film breaking operations of the multi-channel multi-assay parallel pre-processing apparatus, thereby implementing efficient detection of a plurality of detection items while controlling a volume of the multi-channel multi-assay POCT fully automatic chemiluminescence device.

### BRIEF DESCRIPTION OF DRAWINGS

To describe technical solutions in embodiments of this application more clearly, the following briefly describes the accompanying drawings required for describing the embodiments or exemplary technologies. Clearly, the accompanying drawings in the following description show merely some embodiments of this application, and a person of ordinary skill in the art may still derive other drawings from these accompanying drawings without creative efforts.
FIG. 1 is a three-dimensional schematic view of a multi-channel multi-assay POCT fully automatic chemiluminescence device according to an embodiment of this application;
FIG. 2 is another three-dimensional schematic view of a partial multi-channel multi-assay POCT fully automatic chemiluminescence device according to an embodiment of this application;
FIG. 3 is another three-dimensional schematic view of a partial multi-channel multi-assay POCT fully automatic chemiluminescence device according to an embodiment of this application;
FIG. 4 is a schematic structural view of a multi-assay reagent strip according to an embodiment of this application;
FIG. 5 is a schematic cross-sectional view of a multi-assay reagent strip according to an embodiment of this application;
FIG. 6 is a three-dimensional schematic view of a multi-channel multi-assay parallel pre-processing apparatus according to an embodiment of this application;
FIG. 7 is another three-dimensional schematic view of a multi-channel multi-assay parallel pre-processing apparatus according to an embodiment of this application;
FIG. 8 is a schematic three-dimensional exploded view of a multi-channel multi-assay parallel pre-processing apparatus according to an embodiment of this application;
FIG. 9 is a schematic structural view of a multi-channel multi-assay parallel pre-processing apparatus according to an embodiment of this application;
FIG. 10 is a three-dimensional schematic view of a gantry according to an embodiment of this application;
FIG. 11 is another three-dimensional schematic view of a gantry according to an embodiment of this application;
FIG. 12 is a three-dimensional schematic view of a specimen pre-processing mechanism according to an embodiment of this application;
FIG. 13 is a three-dimensional schematic view of a specimen extraction mechanism according to an embodiment of this application;
FIG. 14 is a three-dimensional schematic view of a reaction chamber body according to an embodiment of this application;
FIG. 15 is a three-dimensional schematic view of a film breaking assembly and a PMT assembly according to an embodiment of this application; and
FIG. 16 is a three-dimensional schematic view of a PMT assembly according to an embodiment of this application.

In the figures:
multi-channel multi-assay POCT fully automatic chemiluminescence device-100;
mounting frame-10;
multi-assay reagent strip-20; reagent strip body-21; liquid sealing region-211; dedicated liquid region-2111; common liquid region-2112; handle-22; specimen well-23; tip head-24; disposable magnetic separation sleeve-25;
reaction chamber assembly-30; guide rail-31; reaction chamber body-32; accommodation groove-33; fourth driving element-34; mounting seat-35; first driven pulley-36;
multi-channel multi-assay parallel pre-processing apparatus-40; gantry-41; first driving element-411; movable space-412; transmission plate-413; first slide rail-414; second slide rail-415; specimen extraction mechanism-42; second mounting frame-421; second slide seat-4211; third driving element-422; extraction assembly-423; injection device-4231; second loading head-4232; second push plate-4233; piston rod-4234; push rod-4235; elastic element-4236; separation plate-4237; specimen pre-processing mechanism-43; first mounting frame-431; first slide seat-4311; second driving element-432; pre-processing assembly-433; magnetic rod-4331; magnetic rod device-4332; first loading head-4333; first push plate-4334; connecting rod-4335;
film breaking assembly-50; seventh driving element-51; fifth mounting frame-52; film breaking head-53;
PMT assembly-60; mounting bracket-61; third mounting frame-62; fourth mounting frame-63; PMT module-64; fifth driving element-65; sixth driving element-66; second driven pulley-67; and second synchronous belt pressing plate-68.

### DESCRIPTION OF EMBODIMENTS

To make the objectives, technical solutions, and advantages of this application clearer, the following further describes this application in detail with reference to the accompanying drawings and embodiments. It should be understood that specific embodiments described herein are merely used to explain this application, but are not intended to limit this application.

It should be noted that when a component is referred to as being "fastened to" or "disposed on" another component, the component may be directly on the another component, or indirectly on the another component. When one component is referred to as being "connected to" another component, the component may be directly or indirectly connected to the another component. Orientation or position relationships indicated by terms such as "top", "bottom", "left", and "right" are orientation or position relationships shown based on the accompanying drawings, and are merely for convenience of description, rather than indicating or implying that a referred apparatus or element must have a specific orientation, and be constructed and operated in a specific orientation. Therefore, the terms should not be construed as a limitation on this application. For a person of ordinary skill in the art, specific meanings of the foregoing terms may be understood according to specific circumstances. Terms "first" and "second" are merely used for description purposes, and should not be understood as indicating or implying relative importance or implicitly indicating a quantity of technical features indicated. The term "a plurality of" means two or more, unless otherwise explicitly and specifically defined.

To describe the technical solutions provided in this application, a detailed description is given below in conjunction with the accompanying drawings and the embodiments.

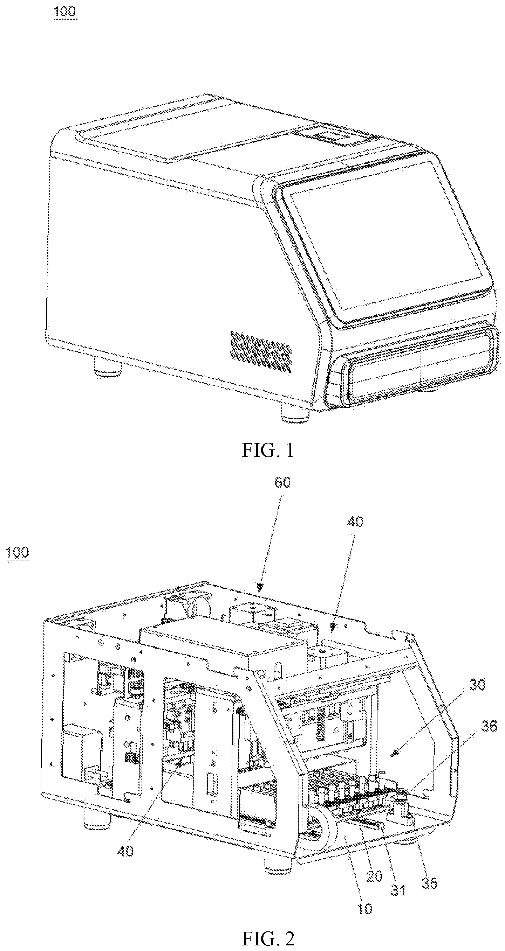

Refer to FIG. 1 to FIG. 7 and FIG. 12. A multi-channel multi-assay POCT fully automatic chemiluminescence device 100 according to an embodiment of this application includes:
a mounting frame 10;
a reaction chamber assembly 30 disposed on the mounting frame 10 and configured to load multi-assay reagent strips 20, where the reaction chamber assembly 30 is reciprocally movable in a length direction of the mounting frame 10, the multi-assay reagent strip 20 is configured to detect at least two markers, the multi-assay reagent strip 20 includes a liquid sealing region 211, and the liquid sealing region 211 includes a common liquid region 2112 and a dedicated liquid region 2111 that are distributed in a length direction of the multi-assay reagent strip 20;
a multi-channel multi-assay parallel pre-processing apparatus 40 disposed on the mounting frame 10 and located above the reaction chamber assembly 30, where the multi-channel multi-assay parallel pre-processing apparatus 40 includes at least two rows of magnetic rods 4331 arranged side by side in a movement direction of the reaction chamber assembly 30, each row of magnetic rods 4331 includes at least two magnetic rods, and the at least two rows of magnetic rods 4331 are configured to transfer magnetic particles in adjacent well positions of the multi-assay reagent strip 20 in the liquid sealing region 211;
a film breaking assembly 50 disposed on the mounting frame 10 and located at a rear end of the multi-channel multi-assay parallel pre-processing apparatus 40, where the film breaking assembly 50 is configured to break a sealing film of the common liquid region 2112; and
a PMT assembly 60 disposed on the mounting frame 10 and located at a rear end of the film breaking assembly 50 and above the reaction chamber assembly 30, where the reaction chamber assembly 30 is configured to move the multi-assay reagent strip 20 to the PMT assembly 60, and the PMT assembly 60 is configured to detect a luminescence value of a detection position of the multi-assay reagent strip 20.

In the multi-channel multi-assay POCT fully automatic chemiluminescence device 100 according to this embodiment of this application, the multi-channel multi-assay parallel pre-processing apparatus 40 includes the at least two rows of magnetic rods 4331 for transferring the magnetic particles, and each row of magnetic rods 4331 includes the at least two magnetic rods. That is, the multi-channel multi-assay parallel pre-processing apparatus 40 may simultaneously transfer the magnetic particles in at least two adjacent reagent wells on a single multi-assay reagent strip 20, and transfer the magnetic particles in adjacent reagent wells of at least two multi-assay reagent strips 20 arranged in parallel. In this way, the at least two markers can be analyzed and detected, and multiple assays can be implemented while achieving multi-channel parallel processing. This can reduce the risk of cross-contamination and shorten the time for transferring the magnetic particles, thereby ensuring the analysis efficiency of the device.

Further, based on a structure of the device and combined with a dedicated design of the multi-assay reagent strip 20, an operation of breaking a sealing film of the multi-assay reagent strip 20 does not need to be completely performed by the multi-channel multi-assay parallel pre-processing apparatus 40. Instead, the sealing film of the common liquid region 2112 may be broken by the film breaking assembly 50 at the rear end. The reaction chamber assembly 30 can implement targeted film breaking without moving the multi-assay reagent strip 20 for a long distance. This reduces a repeated and long-distance movement of the reaction chamber assembly 20, and reduces repeated film breaking operations of the multi-channel multi-assay parallel pre-processing apparatus 40, thereby implementing efficient detection of a plurality of detection items while controlling a volume of the multi-channel multi-assay POCT fully automatic chemiluminescence device 100.

Specifically, the multi-channel multi-assay POCT fully automatic chemiluminescence device 100 in this embodiment of this application is generally rectangular, and the mounting frame 10 is a basic structure of the multi-channel multi-assay POCT fully automatic chemiluminescence device 100. The mounting frame 10 may include a bottom plate, a plurality of side plates disposed on the bottom plate, a top plate, and the like. The mounting frame 10 enclosed by the bottom plate, the plurality of side plates, and the top plate is also generally rectangular.

The mounting frame 10 is configured to carry and mount the foregoing components such as the reaction chamber assembly 30, the multi-channel multi-assay parallel pre-processing apparatus 40, the film breaking assembly 50, and the PMT assembly 60. The foregoing components such as the reaction chamber assembly 30, the multi-channel multi-assay parallel pre-processing apparatus 40, the film breaking assembly 50, and the PMT assembly 60 are disposed in a space of the mounting frame 10. The mounting frame 10 provides stable support for the foregoing components and protects the foregoing components from interference from external impurities, water vapor, and the like.

A plurality of accommodation grooves 33 arranged in parallel may be formed in the reaction chamber assembly 30. The accommodation grooves 33 extend in a length direction of the reaction chamber assembly 30. The multi-assay reagent strips 20 are loaded in the accommodation grooves 33. The plurality of accommodation grooves 33 may accommodate the plurality of multi-assay reagent strips 20, that is, the reaction chamber assembly 30 may drive the plurality of multi-assay reagent strips 20 to move in the multi-channel multi-assay POCT fully automatic chemiluminescence device 100, so that the multi-channel multi-assay POCT fully automatic chemiluminescence device 100 can sequentially perform multi-channel detection and analysis on the plurality of multi-assay reagent strips.

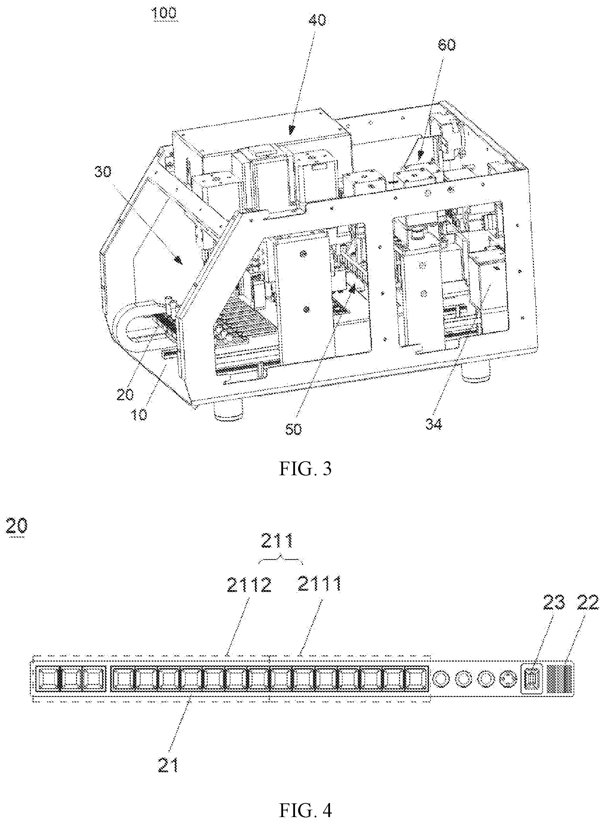

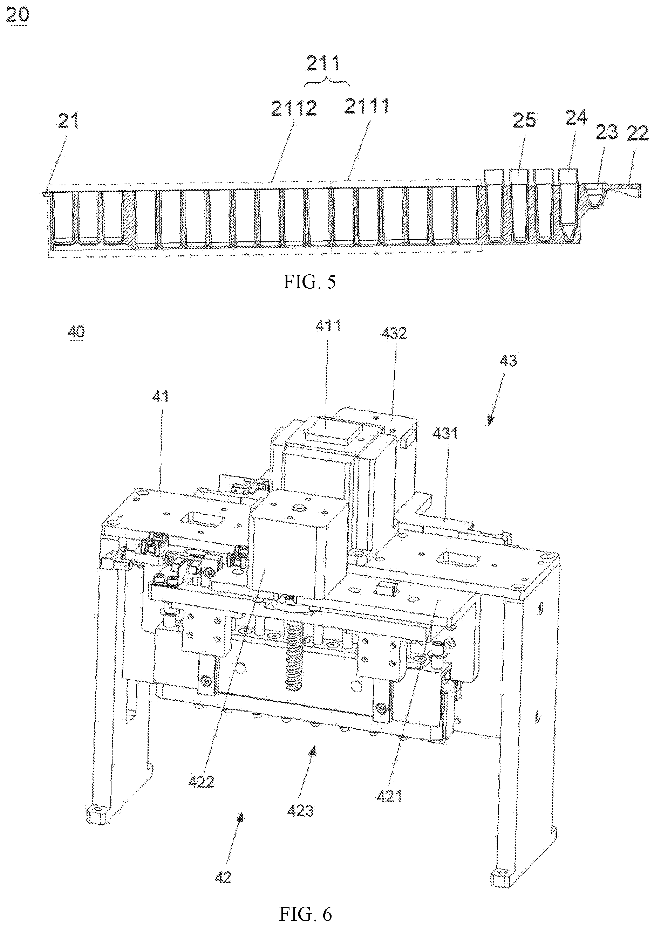

Refer to FIG. 4 and FIG. 5. Further, the multi-assay reagent strip 20 includes:
a reagent strip body 21, where the liquid sealing region 211 is disposed on the reagent strip body 21;
a handle 22 disposed at one end of the reagent strip body 21; and
a specimen well 23, a tip head 24, at least two disposable magnetic separation sleeves 25, the dedicated liquid region 2111, and the common liquid region 2112, which are disposed on the reagent strip body 21 and sequentially arranged from the handle 22 to the other end of the reagent strip body 21.

The dedicated liquid region 2111 includes at least two carbon particle-labeled ligands and at least two enzyme-labeled ligands, and the common liquid region 2112 includes a cleaning solution and a luminescent substrate solution.

In this embodiment of this application, the multi-assay reagent strip 20 is a disposable consumable and may be directly discarded and destroyed after use. The multi-assay reagent strip 20 is generally strip-shaped, the handle 22 may be disposed at one end of the multi-assay reagent strip 20, and anti-slip patterns may be disposed on the handle 22 to increase friction between the multi-assay reagent strip 20 and fingers of an operator, facilitating a firm grip by the operator.

The specimen well 23, a tip head well, at least two disposable magnetic separation sleeve wells, at least two magnetic-labeled ligand wells, at least two enzyme-labeled ligand wells, a plurality of cleaning solution wells, and a plurality of substrate reading wells (i.e., the detection positions described above) are sequentially disposed in a direction from one end of the handle 22 to the other end of the multi-assay reagent strip 20. The magnetic-labeled ligand wells and the enzyme-labeled ligand wells constitute the dedicated liquid region 2111, the cleaning solution wells and the substrate reading wells constitute the common liquid region 2112, and the dedicated liquid region 2111 and the common liquid region 2112 constitute the liquid sealing region 211.

The specimen well 23 is configured to add a blood specimen, the tip head 24 is disposed in the tip head well, the disposable magnetic separation sleeve 25 is disposed in the disposable magnetic separation sleeve well, the magnetic-labeled ligand is accommodated in the magnetic-labeled ligand well, and the enzyme-labeled ligand is accommodated in the enzyme-labeled ligand well, where the ligand includes, but is not limited to, a hapten, an antigen, a monoclonal antibody, a polyclonal antibody, and a nucleic acid ligand. The cleaning solution is accommodated in the cleaning solution well, and a substrate reading reagent is accommodated in the substrate reading well. The number of rows of the magnetic rods 4331 is equal to the number of the disposable magnetic separation sleeve wells, the magnetic-labeled ligand wells, the enzyme-labeled ligand wells, and the substrate reading wells.

In addition, after the corresponding reagents are encapsulated in the corresponding well positions of the multi-assay reagent strip 20, a surface of the multi-assay reagent strip 20 may be heat-sealed with a film, so as to prevent leakage or contamination of the reagent in each well position and facilitate storage and transportation of the multi-assay reagent strip 20.

The tip head 24 is of a hollow structure with a sharp end portion, and a through hole is disposed on the end portion, so that the tip head 24 may be configured to transfer specimens or reagents, and may also be configured to break the sealing film on each well position. The disposable magnetic separation sleeve 25 may be configured to adsorb, mix, clean, and transfer the magnetic particles. The cleaning solution well may be configured to clean the magnetic particles. The substrate reading well is configured to perform a chemiluminescence reaction and reading the luminescence value.

In this embodiment of this application, the dedicated liquid region 2111 may be understood as components directly related to the detection items (for example, magnetic bead-labeled ligands, the enzyme-labeled ligands, and pre-processing solutions), where the ligands include a hapten, an antigen, an antibody, a nucleic acid probe, and the like, and the common liquid region 2112 may be understood as components that may be shared among the detection items (for example, the cleaning solution and a luminescent solution).

In the common liquid region 2112, even if the film breaking assembly 50 is contaminated with liquid from one well position during a previous film breaking process and then other well positions are contaminated with the liquid, detection results will not be significantly affected. Therefore, the film breaking assembly 50 is designed in this embodiment of this application to specifically break the sealing film of the common liquid region 2112, reduce the film breaking operations of the multi-channel multi-assay parallel pre-processing apparatus 40, shorten a movement distance of the reaction chamber assembly 30, control the device volume, and improve the multi-item analysis efficiency of the device.

Since the reaction chamber assembly 30 is disposed on the bottom plate of the mounting frame 10, the PMT assembly 60 may be disposed above the bottom plate through a structure such as a bracket, that is, the PMT assembly 60 is located relatively above the reaction chamber assembly 30. To ensure that the PMT assembly 60 can detect the luminescence values of all the multi-assay reagent strips 20 in the reaction chamber assembly 30, the PMT assembly 60 may move up, down, left, and right in the mounting frame 10, that is, the PMT assembly 60 may move in a vertical direction and a horizontal direction (a width direction of the mounting frame 10), so as to approach and move away from the multi-assay reagent strips 20, and move and switch back and forth between the plurality of multi-assay reagent strips 20.

Similarly, the multi-channel multi-assay parallel pre-processing apparatus 40 may also be disposed above the bottom plate through a structure such as a bracket, that is, the multi-channel multi-assay parallel pre-processing apparatus 40 is located relatively above the reaction chamber assembly 30. To ensure that the multi-channel multi-assay parallel pre-processing apparatus 40 can pre-process all the multi-assay reagent strips 20 in the reaction chamber assembly 30, the multi-channel multi-assay parallel pre-processing apparatus 40 may also move up and down in the mounting frame 10, that is, the multi-channel multi-assay parallel pre-processing apparatus 40 may move in the vertical direction, so as to approach and move away from the multi-assay reagent strips 20 to transfer specimens or reagents, break the sealing film on the well positions of the multi-assay reagent strips 20, and adsorb, mix, clean, and transfer the magnetic particles, and so on.

In this embodiment of this application, the multi-channel multi-assay parallel pre-processing apparatus 40 may include two parts of structures: one structure is configured to combine with the tip head 24 to transfer the specimens or reagents and break the sealing film of each well position, and the other structure is configured to combine with the disposable magnetic separation sleeves 25 to adsorb, mix, clean, and transfer the magnetic particles.

Refer to FIG. 6 to FIG. 13. Further, the multi-channel multi-assay parallel pre-processing apparatus 40 includes:
a gantry 41 disposed on the bottom plate of the mounting frame 10, where the top of the gantry 41 is provided with a first driving element 411;
a specimen extraction mechanism 42 disposed on the gantry 41, where the specimen extraction mechanism 42 is in transmission connection to the first driving element 411 and may ascend and descend under the drive of the first driving element 411 to aspirate and dispense the specimens on the multi-assay reagent strips 20; and
a specimen pre-processing mechanism 43 disposed on the gantry 41, where the specimen pre-processing mechanism 43 is in transmission connection to the first driving element 411 and may ascend and descend under the drive of the first driving element 411, and the at least two rows of magnetic rods 4331 are disposed on the specimen pre-processing mechanism 43 to transfer the magnetic particles on the multi-assay reagent strips 20.

Specifically, the gantry 41 may be made of metal materials, such as stainless steel, iron, aluminum, and various alloys, and has high strength, which can extend the service life of the multi-channel pre-processing apparatus 40. The gantry 41 spans across the width direction of the mounting frame 10, so that the specimen extraction mechanism 42 and the specimen pre-processing mechanism 43 can span above the reaction chamber assembly 30 to implement multi-channel parallel pre-processing.

In this embodiment of this application, the first driving element 411 may be a lead screw motor, which has high movement precision and is more accurate and stable for position adjustment. The first driving element 411 disposed on the top of the gantry 41 drives the specimen extraction mechanism 42 and the specimen pre-processing mechanism 43 to ascend and descend on the gantry 41, so that the specimen extraction mechanism 42 and the specimen pre-processing mechanism 43 approach or move away from the multi-assay reagent strip 20, thereby allowing the specimen extraction mechanism 42 to cooperate with the tip head 24 on the multi-assay reagent strip 20 to implement functions such as film breaking, specimen transfer, or reagent transfer, and the specimen pre-processing mechanism 43 to cooperate with the disposable magnetic separation sleeves 25 on the multi-assay reagent strip 20 to implement adsorption, mixing, cleaning, and transfer of the magnetic particles.

In addition, the specimen pre-processing mechanism 43 and the specimen extraction mechanism 42 are respectively located on two opposite sides of the gantry 41. For example, the specimen pre-processing mechanism 43 is located on one side of the gantry 41 close to the PMT assembly 60, and the specimen extraction mechanism 42 is located on the other side of the gantry 41.

Accordingly, this can avoid problems of inconvenient operations and structural instability of the gantry 41 caused by the arrangement of the specimen pre-processing mechanism 43 and the specimen extraction mechanism 42 on the same side of the gantry 41, resulting in crowded space and a heavy load on one side of the gantry 41. The specimen extraction mechanism 42 and the specimen pre-processing mechanism 43 may also correspond to reagent wells with different functions on the multi-assay reagent strip 20. For example, a front section of the multi-assay reagent strip 20 requires more participation of the specimen extraction mechanism 42, while a rear section requires more participation of the specimen pre-processing mechanism 43, which can further improve a pre-processing speed of the multi-channel multi-assay parallel pre-processing apparatus 40 for the multi-assay reagent strip 20.

Refer to FIG. 6 to FIG. 12. Further, the specimen pre-processing mechanism 43 includes:
a first mounting frame 431 disposed on the gantry 41 and being in transmission connection to the first driving element 411, where the first driving element 411 may drive the first mounting frame 431 to ascend and descend;
a second driving element 432 disposed on the first mounting frame 431; and
a pre-processing assembly 433 disposed on the first mounting frame 431 and being in transmission connection to the second driving element 432, where the second driving element 432 may drive the pre-processing assembly 433 to act in the vertical direction to transfer the magnetic particles.

Specifically, the first driving element 411 may drive the first mounting frame 431 to ascend and descend, so as to drive the specimen pre-processing mechanism 43 to ascend and descend, to perform corresponding processing such as transferring the magnetic particles on the multi-assay reagent strip 20. The first mounting frame 431 may specifically be generally in an "L" shape, and includes two parts of structures, such as a horizontally extending plate and a vertically extending plate. The second driving element 432 may be disposed on the horizontally extending plate to ensure stable arrangement, so as to provide stable power for the lifting of the pre-processing assembly 433.

The pre-processing assembly 433 is disposed on the vertically extending plate. The second driving element 432 may be a lead screw motor. The lead screw motor has high movement precision, and is more accurate and stable for position adjustment. A transmission shaft (i.e., a transmission lead screw) extends downward after passing through the horizontally extending plate to be in transmission connection to the pre-processing assembly 433. The transmission connection therebetween may be a threaded connection, which can ensure the connection stability therebetween. In addition, the second driving element 432 may drive the pre-processing assembly 433 to act stably and accurately in the vertical direction in a threaded driving manner, so as to stably and accurately adsorb and then transfer the magnetic particles.

Further, to ensure the stable lifting of the pre-processing assembly 433 on the first mounting frame 431, a slide rail extending in the vertical direction may be disposed on the first mounting frame 431, and a slider matching the slide rail may be disposed on a first push plate 4334, so as to implement the stable movement of the pre-processing assembly 433 on the first mounting frame 431 through cooperation between the slide rail and the slider.

Further, a first detector (for example, a photoelectric switch) may be disposed on one side of the top of the gantry 41, and a first sensing member (for example, a baffle) may be disposed on one side of the top of the first mounting frame 431. The first sensing member may generate induction or disconnect induction with the first detector as the first mounting frame 431 ascends and descends. When the first mounting frame 431 drives the first sensing member to move to the first detector to disconnect the induction, it indicates that the specimen pre-processing mechanism 43 moves to a set height. In this case, the power supply to the first driving element 411 may be disconnected to avoid overload damage to the first driving element 411 and reduce power consumption.

Further, a first slide seat 4311 may be disposed on one side of the first mounting frame 431 towards the gantry 41, and a first slide rail 414 extending in a height direction of the gantry 41 may be disposed on one side of the gantry 41 towards the first mounting frame 431. The first slide seat 4311 is slidably connected to the first slide rail 414.

Moreover, the first slide seats 4311 and the first slide rails 414 are provided as two sets, which are disposed on left and right sides of the gantry 41 and the first mounting bracket 431, respectively. Through the cooperation between the two sets of first slide seats 4311 and first slide rails 414, the smooth lifting of the first mounting bracket 431 on the gantry 41 under the drive of the first driving element 411 can be ensured, thereby ensuring the smooth and accurate lifting of the specimen extraction mechanism 42.

In this embodiment of this application, the specimen extraction mechanism 42 is opposite to the specimen pre-processing mechanism 43 on the gantry 41, and the specimen extraction mechanism 42 is located in front of the specimen pre-processing mechanism 43. The first driving element 411, the second driving element 432, and a third driving element 422 are linearly arranged on the top of the gantry 41, so that a top structure of the multi-channel multi-assay parallel pre-processing apparatus 40 is regularly distributed, and the three driving elements are basically concentrated in a middle position of the gantry 41 when exerting force, and the gantry 41 is uniformly stressed and is not prone to shaking and displacement.

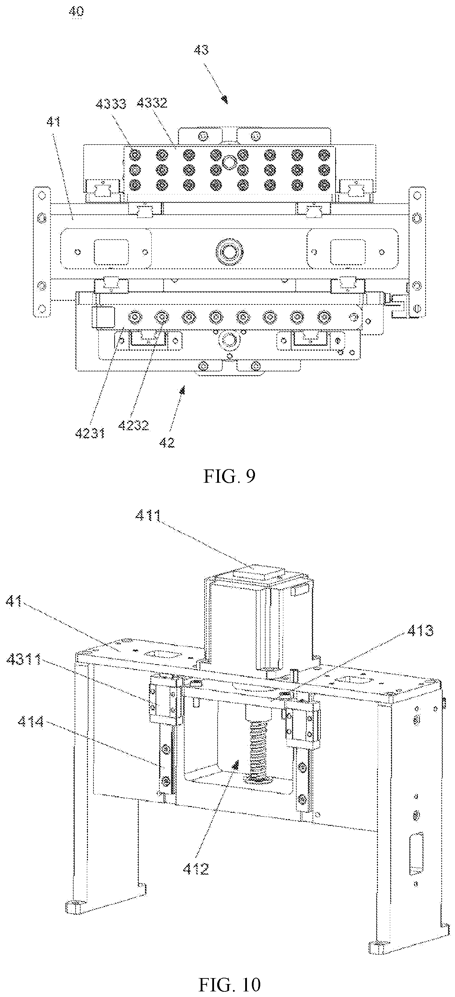

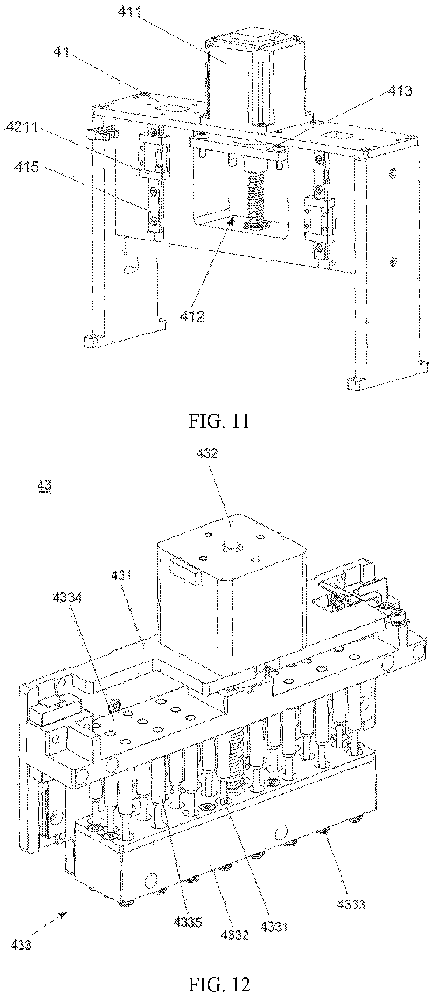

Refer to FIG. 9 and FIG. 12. Further, the pre-processing assembly 433 includes:
a magnetic rod device 4332 disposed on the first mounting frame 431, where a plurality of movable through holes are formed in the magnetic rod device 4332, first loading heads 4333 for loading the disposable magnetic separation sleeves 25 are disposed at the bottom of the magnetic rod device 4332, and the first loading heads 4333 communicate with the movable through holes;
a first push plate 4334 disposed above the magnetic rod device 4332, where the first push plate 4334 is in transmission connection to the second driving element 432; and
a plurality of connecting rods 4335 disposed on the first push plate 4334, where the at least two rows of magnetic rods 4331 are disposed at free ends of the plurality of connecting rods 4335 in a one-to-one manner, and the second driving element 432 may drive the first push plate 4334 to drive the magnetic rods 4331 to ascend and descend in the corresponding movable through holes, so as to be spaced apart from or pass through the first loading heads 4333.

Specifically, the magnetic rod device 4332 is generally cuboid and is distributed in a width direction of the first mounting frame 431, so that the movable through holes therein can be distributed in a width direction of the pre-processing assembly 433, so as to correspond to a location of the multi-assay reagent strip 20.

When the disposable magnetic separation sleeve 25 needs to be loaded, the first driving element 411 drives the first mounting frame 431 to descend until the first loading head 4333 makes contact with the disposable magnetic separation sleeve 25 for loading, and an interference fit is formed therebetween, so as to ensure the stability of the disposable magnetic separation sleeve 25 loaded on the first loading head 4333 and facilitate separation. The first loading head 4333 communicates with the movable through hole, so that the movable through hole communicates with the disposable magnetic separation sleeve 25. When the second driving element 432 drives the connecting rod 4335 to drive the magnetic rod 4331 to pass through the first loading head 4333 to extend into the disposable magnetic separation sleeve 25, the magnetic particles may be adsorbed by the disposable magnetic separation sleeve 25.

When the disposable magnetic separation sleeve 25 needs to be separated, the second driving element 432 is controlled to drive the magnetic rod 4331 to descend and abut against a bottom end of the disposable magnetic separation sleeve 25, so that the disposable magnetic separation sleeve 25 may be removed from the first loading head 4333. When the second driving element 432 drives the connecting rod 4335 to drive the magnetic rod 4331 to separate from the disposable magnetic separation sleeve 25 with an interval from the first loading head 4333, a magnetic field generated by the magnetic rod 4331 disappears, and the carbon particles can be separated from the disposable magnetic separation sleeve 25 to be transferred.

Based on the design of the at least two rows of magnetic rods 4331 in the pre-processing assembly 433, the pre-processing assembly 433 can load at least two rows of disposable magnetic separation sleeves 25 at a time, and the magnetic particles in at least two reagent wells can be transferred at a time through the at least two rows of disposable magnetic separation sleeves 25. This can not only improve a transfer speed of the magnetic particles to ensure the analysis efficiency of the device, but also implement a multi-assay function of the device.

In addition, on the basis of transferring the magnetic particles, the pre-processing assembly 433 combined with the disposable magnetic separation sleeve 25 may also be configured to transfer the magnetic particles to the reagent for a reaction and mixing, or to separate the magnetic particles in a reaction solution into the cleaning solution, or to transfer the magnetic particles to the substrate reading reagent to perform a reaction and complete luminescence detection.

The transmission shaft of the second driving element 432 extends downward through the top of the first mounting frame 431 and the first push plate 4334. A fastener which is in threaded connection to the transmission shaft may be disposed on the transmission shaft, and the first push plate 4334 is fastened to the fastener. When the transmission shaft of the second driving element 432 acts, the first push plate 4334 may be driven to ascend and descend by the fastener to implement the lifting of the magnetic rod 4331.

Alternatively, a threaded hole may also be directly formed in the first push plate 4334, and threads are disposed on the transmission shaft of the second driving element 432. The transmission shaft is in threaded connection to the first push plate 4334 (threaded hole). This can not only ensure the connection stability therebetween, but also allow the second driving element 432 to drive the first push plate 4334 to ascend and descend stably in the threaded driving manner.

Refer to FIG. 9 and FIG. 12. Further, the magnetic rods 4331 are arranged in a plurality of rows, and the plurality of rows of magnetic rods 4331 are evenly spaced in a width direction of the first push plate 4334. The number of rows of the magnetic rods 4331 is equal to the number of the substrate reading wells, so as to implement the detection of the plurality of items, that is, to implement multiple assays.

Each row of magnetic rods 4331 includes a plurality of magnetic rods, and the plurality of magnetic rods 4331 in each row are evenly spaced in a length direction of the first push plate 4334. The number of the magnetic rods 4331 in each row is equal to the number of the multi-assay reagent strips 20, so as to implement multi-channel parallel processing of the plurality of multi-assay reagent strips 20.

In this embodiment of this application, the magnetic rods 4331 are arranged in three rows, which can implement the detection of three items. A space occupied by the three rows of magnetic rods 4331 is not too large, and three-item detection can be implemented, which can implement the multiple assays while controlling the volume of the multi-channel multi-assay POCT fully automatic chemiluminescence device 100.

Certainly, in other embodiments, the magnetic rods 4331 may also be arranged in two, four, or even more rows, so as to implement the detection of different numbers of items.

The specimen pre-processing mechanism 43 in this embodiment of this application may perform reciprocal magnetic adsorption through the lifting drive of the magnetic rod 4331 by the second driving element 432, so that the efficiency of magnetic separation is higher, and the multi-channel parallel separation processing further improves the separation and transfer efficiency of the magnetic particles. In addition, by implementing a reciprocal movement of the disposable magnetic separation sleeve 25 through the second driving element 432, the mixing of the reaction solution, the re-suspension in the cleaning solution, and the like may also be completed.

For example, a process of transferring the magnetic particles by the specimen pre-processing mechanism 43 is generally as follows:
after the specimen is injected into the corresponding reagent well of the multi-assay reagent strip 20, the reaction chamber assembly 30 drives the multi-assay reagent strip 20 to move to a corresponding position, so that the specimen pre-processing mechanism 43 is located directly above a sleeve of the disposable magnetic rod 4331. In this case, the first driving element 411 drives the first mounting frame 431 to descend by a certain height, until the sleeve of the disposable magnetic rod 4331 is loaded on the first loading head 4333, the first driving element 411 drives the first mounting frame 431 to ascend, and the sleeve of the disposable magnetic rod 4331 is driven by the first loading head 4333 to ascend to a certain height.

After that, the reaction chamber assembly 30 moves until the specimen pre-processing assembly 433 loaded with the sleeve of the disposable magnetic rod 4331 moves above the corresponding well position of the multi-assay reagent strip 20, and the second driving element 432 drives the magnetic rod 4331 to descend until the magnetic rod 4331 makes contact with the bottom of the disposable magnetic separation sleeve 25. In this case, the first driving element 411 drives the first mounting frame 431 to descend by a certain height, so that the sleeve of the disposable magnetic rod 4331 extends into liquid containing the carbon particles (for example, the magnetic-labeled ligand mentioned above), until the sleeve of the disposable magnetic rod 4331 makes contact with the bottom of the magnetic-labeled ligand well, so that the magnetic particles are gradually adsorbed to an end portion of the disposable magnetic separation sleeve 25, and the adsorption of the magnetic particles may be fully completed by a plurality of slow ascents and descents.

After the adsorption of the magnetic particles is completed, the first driving element 411 is controlled to drive the first mounting frame 431 to ascend. In this case, all the magnetic particles are adsorbed on an outer wall of the disposable magnetic separation sleeve 25. The reaction chamber assembly 30 is controlled to move until the disposable magnetic separation sleeve 25 is located above the cleaning solution. The first driving element 411 drives the first mounting frame 431 to descend until the disposable magnetic separation sleeve 25 extends into the cleaning solution. After that, the second driving element 432 drives the magnetic rod 4331 to ascend from the disposable magnetic separation sleeve 25, so that the magnetic field disappears, and the magnetic particles are separated and detached from the outer wall of the disposable magnetic separation sleeve 25 and enter the cleaning solution.

To accelerate the separation process of the magnetic particles, the first driving element 411 may drive the first mounting frame 431 to repeatedly ascend and descend at different frequencies, so that the magnetic particles repeatedly act in the cleaning solution, thereby ensuring sufficient mixing and suspension of the magnetic particles with the cleaning solution. After the magnetic particles are completely detached from the outer wall of the disposable magnetic separation sleeve 25, the first driving element 411 drives the first mounting frame 431 to ascend to an initial height, completing one-time operation of magnetic particle separation, cleaning, and mixing. If a plurality of magnetic separation and cleaning operations are required, the foregoing operations can be repeated.

Similarly, if the magnetic particles need to be transferred to other well positions, only the first driving element 411 needs to be controlled to drive the first mounting frame 431 to descend until the disposable magnetic separation sleeve 25 extends into the bottom of a corresponding liquid level, and then the second driving element 432 is controlled to drive the magnetic rod 4331 to descend to make contact with a bottom end of the disposable magnetic separation sleeve 25, so that all the magnetic particles are adsorbed on the outer wall of the disposable magnetic separation sleeve 25, and the first driving element 411 is controlled to drive the first mounting frame 431 to ascend.

After that, the reaction chamber assembly 30 is controlled to move until the disposable magnetic separation sleeve 25 with the adsorbed magnetic particles moves directly above a target well position, and then the first driving element 411 is controlled to drive the first mounting frame 431 to descend until the disposable magnetic separation sleeve 25 extends into target liquid. In this case, the second driving element 432 is controlled to drive the magnetic rod 4331 to ascend to make the magnetic field disappear, and the magnetic particles can be gradually separated into the target liquid. The separation of the magnetic particles may be accelerated by controlling the first driving element 411 to repeatedly drive the first mounting frame 431 to ascend and descend, while making the disposable magnetic separation sleeve 25 repeatedly act in the target liquid.

Refer to FIG. 6 to FIG. 11 and FIG. 13. Further, the specimen extraction mechanism 42 includes:
a second mounting frame 421 disposed on the gantry 41 and being in transmission connection to the first driving element 411, where the first driving element 411 may drive the second mounting frame 421 to ascend and descend;
a third driving element 422 disposed on the second mounting frame 421; and
an extraction assembly 423 disposed on the second mounting frame 421 and being in transmission connection to the third driving element 422, where the third driving element 422 may drive the extraction assembly 423 to ascend and descend to aspirate and dispense specimens.

Specifically, the first driving element 411 may drive the second mounting frame 421 to ascend and descend, to drive the specimen extraction mechanism 42 to ascend and descend, so as to perform corresponding processing such as specimen aspirating and dispensing on the multi-assay reagent strip 20. The second mounting frame 421 may specifically be generally in an "L" shape, and includes two parts of structures, such as a horizontally extending plate and a vertically extending plate. The third driving element 422 may be disposed on the horizontally extending plate to ensure stable arrangement, so as to provide stable power for the lifting of the extraction assembly 423.

The extraction assembly 423 is disposed on the vertically extending plate. The third driving element 422 may be a lead screw motor. The lead screw motor has high movement precision, and is more accurate and stable for position adjustment. A transmission shaft (i.e., a transmission lead screw) extends downward after passing through the horizontally extending plate to be in transmission connection to the extraction assembly 423. The transmission connection therebetween may be a threaded connection, which can ensure the connection stability therebetween. In addition, the third driving element 422 may drive the extraction assembly 423 to act stably and accurately in the vertical direction in the threaded driving manner, so as to stably and accurately aspirate and dispense the specimen after making contact with the specimen.

Further, a second detector (for example, a photoelectric switch) may be disposed on one side of the top of the gantry 41, and a second sensing member (for example, a baffle) may be disposed on one side of the top of the second mounting frame 421. The second sensing member may generate induction or disconnect induction with the second detector as the second mounting frame 421 ascends and descends.

When the second mounting frame 421 drives the second sensing member to move to the second detector to disconnect the induction, it indicates that the specimen extraction mechanism 42 moves to a set height. In this case, the power supply to the first driving element 411 may be disconnected to avoid overload damage to the first driving element 411 and reduce power consumption.

Moreover, a second slide seat 4211 may be disposed on one side of the second mounting frame 421 towards the gantry 41, and a second slide rail 415 extending in the height direction of the gantry 41 may be disposed on one side of the gantry 41 towards the second mounting frame 421. The second slide seat 4211 is slidably connected to the second slide rail 415.

Moreover, the second slide seats 4211 and the second slide rails 415 on the second mounting frame 421 and the gantry 41 are provided as two sets, which are disposed on the left and right sides of the gantry 41 and the second mounting frame 421, respectively. In this way, through the cooperation between the two sets of second slide seats 4211 and second slide rails 415, it may be ensured that the first driving element 411 drives the second mounting frame 421 to ascend and descend stably on the gantry 41, thereby ensuring that the specimen extraction mechanism 42 ascends and descends stably and accurately, and ensuring the stable operation of the device.

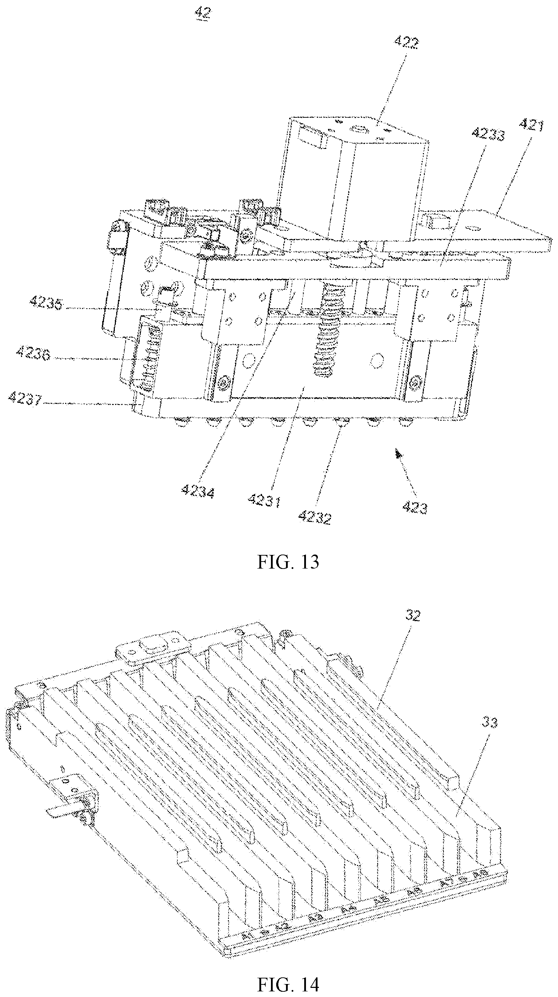

Refer to FIG. 13. Further, the extraction assembly 423 includes:
an injection device 4231 disposed on the second mounting frame 421, where a plurality of injector cavities are formed in the injection device 4231, a plurality of second loading heads 4232 for loading the tip heads 24 are disposed at the bottom of the injection device 4231, and the plurality of second loading heads 4232 communicate with the plurality of injector cavities in a one-to-one manner;
a second push plate 4233 disposed above the injection device 4231, where the second push plate 4233 is in transmission connection to the third driving element 422; and
a plurality of piston rods 4234 disposed on the second push plate 4233, where the piston rods 4234 are movably and sealingly connected to the injector cavities, and the third driving element 422 may drive the second push plate 4233 to drive the piston rods 4234 to ascend and descend in the injector cavities to perform aspirating and dispensing actions.

Specifically, the injection device 4231 is generally cuboid, and is distributed in a width direction of the second mounting frame 421, so that the injector cavities therein can be distributed in a width direction of the extraction assembly 423, so as to correspond to the location of the multi-assay reagent strip 20. The injection device 4231 also has a certain height to ensure that spaces of the injector cavities are large enough, so that the piston rods 4234 can generate a sufficiently large aspirating and dispensing force when moving in the injector cavities.

The second loading head 4232 and the tip head 24 are in an interference fit, so as to ensure the stability of the tip head 24 loaded on the second loading head 4232 and facilitate the removal of the tip head 24 in a pushing manner. When the first driving element 411 drives the second mounting frame 421 to drive the extraction assembly 423 to descend, the corresponding sealing film on the multi-assay reagent strip 20 may be broken by the tip head 24 loaded on the second loading head 4232. The second loading heads 4232 communicate with the injector cavities, so that the injector cavities communicate with the tip heads 24. When the piston rods 4234 are pushed to ascend and descend in the injector cavities, the specimens may be aspirated and dispensed through the tip heads 24.

The transmission shaft of the third driving element 422 extends downward through the top of the second mounting frame 421 and the second push plate 4233. A fastener which is in threaded connection to the transmission shaft may be disposed on the transmission shaft, and the second push plate 4233 is fastened to the fastener. When the transmission shaft of the third driving element 422 acts, the second push plate 4233 may be driven to ascend and descend by the fastener to implement the lifting of the piston rod 4234.

Alternatively, a threaded hole may also be formed in the second push plate 4233, and threads are disposed on the transmission shaft of the third driving element 422. The transmission shaft is in threaded connection to the second push plate 4233. This can not only ensure the connection stability therebetween, but also allow the third driving element 422 to drive the second push plate 4233 to ascend and descend in the threaded driving manner.

In this embodiment, an end portion of the piston rod 4234 in the injector cavity may be sealed with the injector cavity through a sealing ring (for example, a rubber ring or a plastic ring). When the piston rod 4234 is driven upward by the second push plate 4233 to ascend in the injector cavity, a negative pressure is generated in the injector cavity, so that the tip head 24 aspirates the specimen. When the piston rod 4234 is pushed downward by the second push plate 4233 to descend in the injector cavity, a positive pressure is generated in the injector cavity, so that the tip head 24 dispenses the specimen.

Further, in order to ensure that the second push plate 4233 stably drives the piston rod 4234 to ascend and descend, slide rails extending in the vertical direction are disposed on the injection device 4231, and sliders matching the slide rails are disposed on the second push plate 4233. The cooperation between the slide rails and the sliders implements a stable movement of the second push plate 4233 relative to the second mounting frame 421, thereby ensuring stable driving of the piston rod 4234. The slide rails and the sliders are provided as two sets, which are disposed on left and right sides of the specimen extraction mechanism 42, respectively, thereby further ensuring the stable lifting of the second push plate 4233.

Further, a photoelectric switch may be disposed on the top of the second mounting frame 421, and a baffle is disposed on a corresponding side of the second push plate 4233. When the second push plate 4233 drives the baffle to move to the photoelectric switch to make the photoelectric switch disconnect the induction, it indicates that the second push plate 4233 moves to a preset height, that is, it indicates that the piston rod 4234 is reset. In this case, the power supply to the third driving element 422 may be disconnected to avoid overload damage to the third driving element 422.

Refer to FIG. 9 and FIG. 13. In this embodiment of this application, the plurality of injector cavities are evenly spaced in a length direction of the injection device 4231; the plurality of second loading heads 4232 are evenly spaced in the length direction of the injection device 4231; and the plurality of piston rods 4234 are evenly spaced in a length direction of the second push plate 4233. That is, the plurality of piston rods 4234, the plurality of injector cavities, and the plurality of piston rods 4234 are in one-to-one correspondence, thereby implementing multi-channel parallel processing of the plurality of multi-assay reagent strips 20.

Still refer to FIG. 13. Further, the specimen extraction mechanism 42 further includes:
push rods 4235 that penetrate through the injection device 4231 in a liftable manner, where top ends of the push rods 4235 are spaced apart from the second push plate 4233;
elastic elements 4236 sleeving the push rods 4235 located in the injection device 4231, where the elastic elements 4236 are simultaneously stretched or compressed when the push rods 4235 ascend or descend relative to the injection device 4231; and
a separation plate 4237 disposed at the bottom of the injection device 4231, where the second loading heads 4232 pass through the separation plate 4237, bottom ends of the push rods 4235 are connected to the separation plate 4237, and the push rods 4235 may drive the separation plate 4237 to ascend and descend relative to the second loading heads 4232.

In this embodiment of this application, the separation plate 4237 is provided with through holes. A size of the through hole is smaller than a size of a joint between the tip head 24 and the second loading head 4232, so that the tip head 24 loaded on the second loading head 4232 may be removed by the arrangement of the separation plate 4237.

Specifically, since the top end of the push rod 4235 is spaced apart from the second push plate 4233, when the second push plate 4233 is pushed downward by the third driving element 422 by a certain height to make contact with the top end of the push rod 4235, the second push plate 4233 pushes the push rod 4235 to descend in the injection device 4231, thereby pushing the separation plate 4237 located at the bottom of the injection device 4231 to descend and abut against the tip head 24, so as to push the tip head 24 to separate from the second loading head 4232.

After the separation of the tip head 24 is completed, the third driving element 422 stops driving, and the elastic element 4236 may be stretched to generate an upward thrust on the push rod 4235, so as to drive the separation plate 4237 to reset. The compression of the elastic element 4236 may provide a certain buffering force for the descent of the push rod 4235, so as to prevent the tip head 24 from being pressed and damaged due to a high descent speed of the push rod 4235, or to prevent the displacement and shaking of the multi-assay reagent strip 20 due to the tip head 24 being pressed on the multi-assay reagent strip 20.

In this embodiment of this application, two push rods 4235 and two elastic elements 4236 are arranged, which are respectively disposed on two sides of the injection device 4231, so as to ensure stable pushing of the separation plate 4237 by the push rods 4235, thereby ensuring a separation effect of the tip head 24, and ensuring stable resetting of the separation plate 4237 by the elastic elements 4236.

For example, the process of aspirating and dispensing the specimen by the specimen extraction mechanism 42 (and reagent transferring/mixing) is generally as follows:
the reaction chamber assembly 30 drives the multi-assay reagent strip 20 to move to a corresponding position, and the first driving element 411 drives the second mounting frame 421 to descend until the second loading head 4232 is fitted with and fastened to the tip head 24 on the multi-assay reagent strip 20.

After the loading of the tip head 24 is completed, the reaction chamber assembly 30 drives the multi-assay reagent strip 20 to move until the tip head 24 moves above the specimen well 23. In this case, the third driving element 422 first drives the piston rod 4234 to descend by a certain height or descend to the lowest point in the injector cavity. Then, the first driving element 411 drives the second mounting frame 421 to descend by a certain height until a tip of the tip head 24 extends into the specimen, and the third driving element 422 drives the piston rod 4234 to ascend to aspirate the specimen through the tip head 24. Then, the first driving element 411 drives the second mounting frame 421 to ascend, so that the tip head 24 is separated from a specimen liquid level. The reaction chamber assembly 30 drives the multi-assay reagent strip 20 to move until the tip head 24 with the aspirated specimen moves directly above the target well position.

The first driving element 411 drives the second mounting frame 421 to descend to drive the tip head 24 with the aspirated specimen to descend until the tip of the tip head 24 extends below a liquid level of the reagent, and the third driving element 422 drives the piston rod 4234 to descend to inject the specimen in the tip head 24 into the reagent. Then, the first driving element 411 drives the second mounting frame 421 to ascend until the tip head 24 is separated from the reagent, and the third driving element 422 drives the piston rod 4234 to ascend to an original position, thereby implementing the aspiration and dispensing transfer of the specimen. After that, the tip head 24 is returned to the tip head well.

The transfer process of the reagent is the same as the transfer process of the specimen, and details are not described herein again.

If a plurality of liquid transfers are needed, actions of loading the tip head 24 and aspiration and injection can be repeated. In addition, the specimen extraction mechanism 42 may also be configured to mix the reagent. The third driving element 422 is controlled to drive the piston rod 4234 to repeatedly ascend and descend in the injector cavity, so that the tip head 24 repeatedly aspirates and dispenses below the liquid level of the reagent, so that the reagent or specimen that needs to react can be fully mixed and reacted.

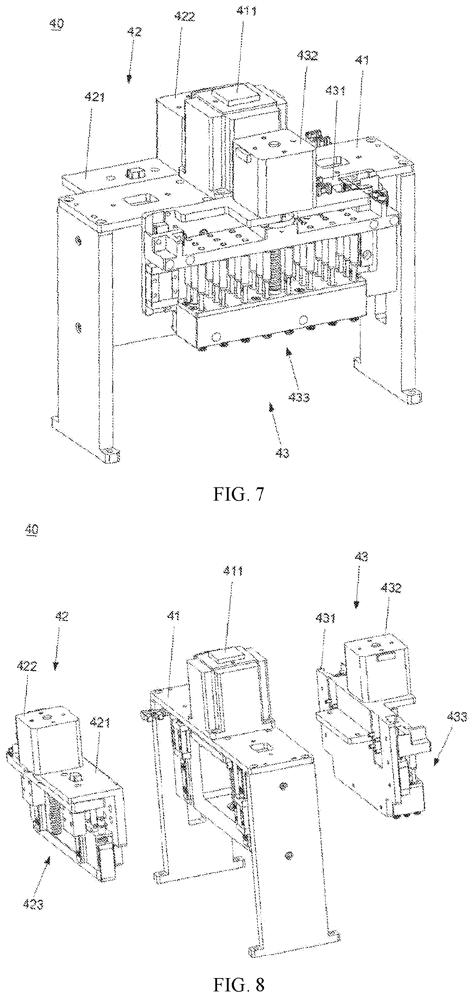

Refer to FIG. 8, FIG. 10 and FIG. 11. Further, the gantry 41 is provided with a movable space 412, the transmission shaft of the first driving element 411 passes through the movable space 412, a transmission plate 413 sleeves the transmission shaft, the transmission shaft is provided with a thread, and the transmission plate 413 is provided with a threaded hole. Therefore, when the transmission shaft acts, the transmission plate 413 can synchronously ascend and descend in the movable space 412 in a threaded transmission manner.

In addition, a first connecting portion is disposed on one side of the first mounting frame 431 towards the gantry 41, and the first connecting portion is formed after the vertically extending plate of the first mounting frame 431 extends towards the gantry 41. A second connecting portion is disposed on one side of the second mounting frame 421 towards the gantry 41, and the second connecting portion is formed after the vertically extending plate of the second mounting frame 421 extends towards the gantry 41. Both the first connecting portion and the second connecting portion are connected to the transmission plate 413 through fasteners (for example, screws). Therefore, when the transmission plate 413 ascends and descends with the transmission shaft of the first driving element 411, the second mounting frame 421 and the first mounting frame 431 can be driven to ascend and descend, that is, the specimen extraction mechanism 42 and the specimen pre-processing mechanism 43 can ascend and descend on the gantry 41.

Refer to FIG. 2, FIG. 3 and FIG. 14. Further, the reaction chamber assembly 30 includes:
a guide rail 31 disposed on the bottom plate of the mounting frame 10;
a reaction chamber body 32 disposed on the guide rail 31, where a guide block that is slidably connected to the guide rail 31 is disposed at the bottom of the reaction chamber body 32, a plurality of accommodation grooves 33 evenly spaced and configured to load the multi-assay reagent strips 20 are disposed on the reaction chamber body 32, and the accommodation grooves 33 extend in a length direction of the reaction chamber body 32;
a fourth driving element 34 disposed on the bottom plate of the mounting frame 10 and located at a rear end of the guide rail 31, where a first driving pulley is disposed on a transmission shaft of the fourth driving element 34;
a mounting seat 35 disposed on the bottom plate of the mounting frame 10 and located at a front end of the guide rail 31, where a first driven pulley 36 is disposed on the mounting seat 35, and the first driving pulley is in transmission connection to the first driven pulley 36 through a belt; and
a first synchronous belt pressing plate disposed on one side of the reaction chamber body 32, where the first synchronous belt pressing plate is connected to the belt.

Specifically, the guide rail 31 extends in a length direction of the mounting frame 10. Through the slidable connection between the guide rail 31 and the guide block, the reaction chamber assembly 30 can stably move in the length direction of the mounting frame 10. A shape of the accommodation groove 33 matches the shape of the multi-assay reagent strip 20, so that the multi-assay reagent strip 20 can be stably loaded on the reaction chamber assembly 30, thereby ensuring stability during movement and pre-processing, avoiding displacement and shaking, and preventing situations such as reagent leakage from the multi-assay reagent strip 20.

The fourth driving element 34 is located at a rear end of the mounting frame 10, and the mounting seat 35 is located at a front end of the mounting frame 10, so that the connection therebetween can cover the length direction of the mounting frame 10, thereby allowing the reaction chamber assembly 30 to achieve a long enough movement distance within the mounting frame 10, so as to better cooperate with the movement of the multi-channel multi-assay parallel pre-processing apparatus 40.

A transmission structure including the first driving pulley, the belt, the first synchronous belt pressing plate, and the first driven pulley 36 has high transmission efficiency, is simple, and has sufficient power. In combination with the arrangement of the guide rail 31, it can be ensured that the reaction chamber assembly 30 moves stably and quickly in the mounting frame 10, thereby improving the movement efficiency of the reaction chamber assembly.

Further, a photoelectric switch for real-time detection of a position of the reaction chamber body 32 may be disposed on the bottom plate of the mounting frame 10, and a baffle may be disposed on the reaction chamber body 32. When the reaction chamber body 32 drives the baffle to move to a corresponding position, causing the baffle to reach the photoelectric switch and the photoelectric switch to disconnect the induction, it indicates that the reaction chamber assembly 30 reaches a set position. In this case, the power supply to the fourth driving element 34 can be disconnected to avoid overload and failure caused by continuous operation of the fourth driving element 34 and reduce power consumption.

In addition, a heating module is also disposed at the bottom of the reaction chamber body 32. A heating temperature of the heating module may be kept constant at a human body temperature, i.e., 37°C. A temperature in the reaction chamber body 32 is kept constant within a preset value range, so that the reagent in the multi-assay reagent strip 20 meets temperature conditions for chemiluminescence measurement.

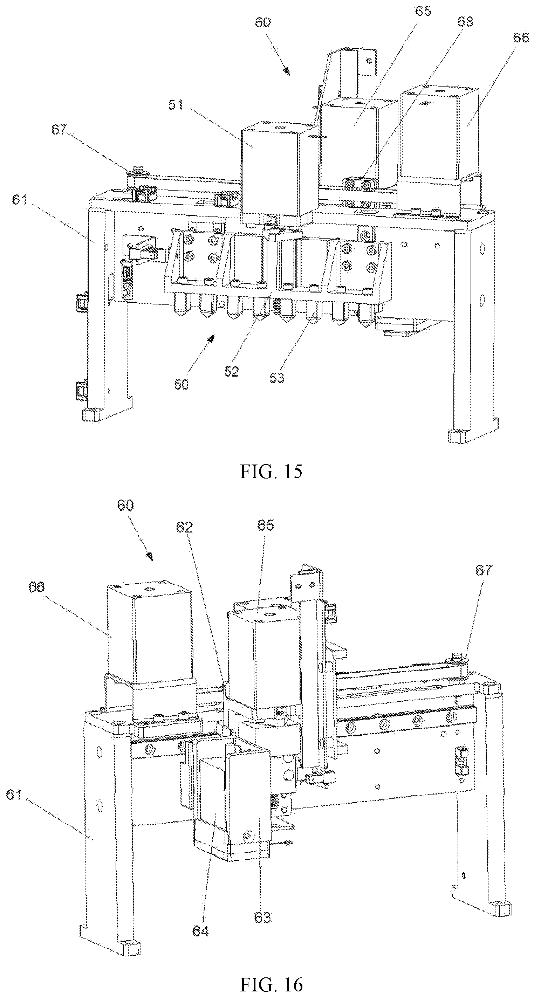

Refer to FIG. 2, FIG. 15, and FIG. 16. Further, the PMT assembly 60 includes:
a mounting bracket 61 disposed on the bottom plate of the mounting frame 10;
a third mounting frame 62 disposed on the mounting bracket 61;
a fourth mounting frame 63 disposed at the bottom of the third mounting frame 62;
a PMT module 64 disposed on the fourth mounting frame 63;
a fifth driving element 65 disposed on the top of the third mounting frame 62 and being in transmission connection to the fourth mounting frame 63, where the fifth driving element 65 may drive the fourth mounting frame 63 to ascend and descend so as to drive the PMT module 64 to ascend and descend;
a sixth driving element 66 and a second driven pulley 67 respectively disposed on two sides of the top of the mounting bracket 61, where a transmission shaft of the sixth driving element 66 is provided with a second driving pulley, and the second driving pulley is in transmission connection to the second driven pulley 67 through a belt; and
a second synchronous belt pressing plate 68 disposed on the third mounting frame 62, where the second synchronous belt pressing plate 68 is connected to the belt.

In this embodiment of this application, a structure of the mounting bracket 61 may be similar to that of the gantry 41: The mounting bracket 61 also spans across the width direction of the mounting frame 10, so that the PMT module 64 can move above the reaction chamber assembly 30 to implement chemiluminescence detection. Moreover, the mounting bracket 61 may also be made of metal materials, such as stainless steel, iron, aluminum, and various alloys, and has high strength, which can extend the service life of the PMT assembly 60.

The third mounting frame 62 is configured to mount the fifth driving element 65, to provide stable support for the fifth driving element 65, so that the fifth driving element 65 is stably located on the top of the mounting bracket 61. The fourth mounting frame 63 is configured to mount the PMT module 64, and is in transmission connection to the fifth driving element 65, so that the PMT module 64 can implement a lifting movement under the drive of the fifth driving element 65. The third mounting frame 62 is in transmission connection to the belt to drive the fourth mounting frame 63 and the PMT module 64 thereon to move in the horizontal direction, so that the PMT module 64 can move up, down, left, and right on the mounting bracket 61 by combining the fifth driving element 65 and the sixth driving element 66.

In this embodiment of this application, the fifth driving element 65 may be a lead screw motor, and the fourth mounting frame 63 is driven to ascend and descend by the fifth driving element 65 disposed on the top of the mounting bracket 61, so as to drive the PMT module 64 to ascend and descend, so that the PMT assembly 60 can approach or move away from the multi-assay reagent strip 20. In addition, the lead screw motor has high movement precision, and is more accurate and stable for position adjustment of the PMT module 64, so that the PMT assembly 60 can accurately and stably detect the luminescence value of the detection position on the multi-assay reagent strip 20.

The sixth driving element 66 may be a stepping motor, which is located on one side (for example, a right side) of the top of the mounting bracket 61, and the second driven pulley 67 is located on the other side (for example, a left side) of the top of the mounting bracket 61, so that the connection therebetween can cover a length direction of the mounting bracket 61, and thus the movement of the PMT module 64 on the mounting bracket 61 can cover a width range of the mounting frame 10.

A transmission structure including the second driving pulley, the belt, the second synchronous belt pressing plate 68, and the second driven pulley 67 has high transmission efficiency, is simple, and has sufficient power. The belt can improve a movement speed of the PMT module 64, thereby improving detection efficiency, so that the PMT assembly 60 can quickly move and switch between the multi-assay reagent strips 20 of the reaction chamber assembly 30 to perform luminescence value detection respectively.

Refer to FIG. 2, FIG. 15 and FIG. 16. Further, the film breaking assembly 50 includes:
a seventh driving element 51 disposed on the top of the mounting bracket 61;
a fifth mounting frame 52 disposed on the mounting bracket 61 and located below the seventh driving element 51, where the fifth mounting frame 52 is in transmission connection to the seventh driving element 51 and can ascend and descend under the drive of the seventh driving element 51; and
a plurality of film breaking heads 53 disposed at the bottom of the fifth mounting frame 52, where the plurality of film breaking heads 53 are evenly spaced in a length direction of the fifth mounting frame 52.

In this embodiment of this application, the film breaking assembly 50 and the PMT assembly 60 share the mounting bracket 61, which can improve a utilization rate of the mounting bracket 61, control an internal volume of the device, and promote miniaturization of the device. The film breaking assembly 50 is located on one side of the mounting bracket 61 close to the multi-channel multi-assay parallel pre-processing apparatus 40, so as to be closer to the multi-assay reagent strip 20, which can shorten a movement distance of the multi-assay reagent strip 20, break the film of the common liquid region 2112 more quickly, and improve the analysis efficiency of the device.

The seventh driving element 51 may be a lead screw motor, and the fifth mounting frame 52 is driven by the seventh driving element 51 disposed on the top of the mounting bracket 61 to ascend and descend, so as to drive the film breaking head 53 to ascend and descend, so that the film breaking head 53 can approach or move away from the multi-assay reagent strip 20, and then break the sealing film through a sharp end portion of the film breaking head 53 and switch film breaking between different reagent wells. Moreover, the lead screw motor has high movement precision and is more accurate and stable for position adjustment of the film breaking head 53, so that the film breaking head 53 can accurately and stably break the sealing film of the common liquid region 2112, and avoid pressing against the multi-assay reagent strip 20 and causing shaking or misalignment.

The number of the film breaking heads 53 is the same as the number of the multi-assay reagent strips 20, so as to break the films of all the multi-assay reagent strips 20 at the same time, improve a processing speed, and then improve the analysis efficiency of the device. Further, the film breaking heads 53 may also be arranged in a plurality of rows. For example, the number of rows is the same as the number of the reagent wells in the common liquid region 2112, so that the film breaking assembly 50 only needs to be operated once to break all the sealing films of the common liquid region 2112, thereby improving the film breaking efficiency and further improving the analysis efficiency of the device.

In this embodiment of this application, a transmission shaft of the seventh driving element 51 extends downward through the top of the fifth mounting frame 52, a fastener which is in threaded connection to the transmission shaft may be disposed on the transmission shaft, and the fifth mounting frame 52 is fastened to the fastener. When the transmission shaft of the seventh driving element 51 acts, the fifth mounting frame 52 may be driven by the fastener to ascend and descend, so as to implement the lifting of the film breaking heads 53.

Alternatively, a threaded hole may also be directly formed in the fifth mounting frame 52, and a thread may be disposed on the transmission shaft of the seventh driving element 51. The transmission shaft is in threaded connection to the fifth mounting frame 52 (the threaded hole), so that the connection stability therebetween can be ensured, and the seventh driving element 51 may also drive the fifth mounting frame 52 to ascend and descend in the threaded driving manner.

In this embodiment of this application, the film breaking head 53 and the fifth mounting frame 52 may be detachably disposed. For example, the film breaking head 53 is disposed on the fifth mounting frame 52 through screws. This can not only ensure the stability of the film breaking head 53 on the fifth mounting frame 52, but also facilitate disassembly and assembly, facilitating replacement when the film breaking head 53 is damaged.

Further, in order to ensure that the seventh driving element 51 stably drives the fifth mounting frame 52 to ascend and descend, slide rails extending in the vertical direction may be disposed on the mounting bracket 61, and sliders matching the slide rails may be disposed on the fifth mounting frame 52. The stable movement of the fifth mounting frame 52 relative to the mounting bracket 61 is implemented through the cooperation between the slide rails and the sliders. Moreover, the slide rails and the sliders are provided as two sets, which are respectively disposed on left and right sides of the mounting bracket 61 and the fifth mounting frame 52, thereby further ensuring the stable lifting of the fifth mounting frame 52.

Further, a photoelectric switch may also be disposed on the top of the mounting bracket 61, and a baffle may be disposed on a corresponding side of the fifth mounting frame 52. When the fifth mounting frame 52 drives the baffle to move to the photoelectric switch to make the photoelectric switch disconnect the induction, it indicates that the fifth mounting frame 52 moves to a set height for resetting. In this case, the power supply to the seventh driving element 51 may be disconnected to avoid overload damage to the seventh driving element 51 and reduce power consumption.

Further, the multi-channel multi-assay POCT fully automatic chemiluminescence device 100 may further include components such as a control module, an electrical module, a display module, an image acquisition module, a heat dissipation module, and an alarm module. The control module is electrically connected to the electrical module, the display module, the image acquisition module, the heat dissipation module, and the alarm module. The electrical module is further electrically connected to the display module, the image acquisition module, the heat dissipation module, and the alarm module.

For example, the electrical module may be configured to implement power support for the entire device, the control module may be configured to implement operation control of the device, such as controlling the opening and closing of each driving element, the opening and closing of the heating module, the display of the display module, and the image acquisition and image conversion of the image acquisition module. The display module may be configured to display data such as operating parameters of the device and item analysis results. The image acquisition module may be configured to shoot and monitor the pre-processing operation of the multi-channel multi-assay parallel pre-processing apparatus 40. When an abnormal operation is monitored, the control module may control the alarm module (for example, sound and light alarm and display alarm) to issue a corresponding warning. The heat dissipation module is configured to dissipate heat for the device to avoid overheating that may affect the normal operation.

The components such as the control module, the electrical module, the display module, the image acquisition module, the heat dissipation module, and the alarm module do not involve inventive points of this application, and thus are not described in detail herein, but only connection relationships and basic functions are briefly described. If there are any unclear points, a person skilled in the art may refer to structures, positions, and functions of the components such as the control module, the electrical module, the display module, the image acquisition module, the heat dissipation module, and the alarm module in the related art.

The foregoing descriptions are merely optional embodiments of this application, and are not intended to limit this application. For a person skilled in the art, various modifications and changes may be made in this application. Any modification, equivalent replacement, improvement, and the like made within the spirit and principle of this application shall fall within the scope of the claims of this application.

## Claims

1. A multi-channel multi-assay POCT fully automatic chemiluminescence device, comprising:
a mounting frame;
a reaction chamber assembly disposed on the mounting frame and configured to load a multi-assay reagent strip, wherein the reaction chamber assembly is reciprocally movable in a length direction of the mounting frame, the multi-assay reagent strip is configured to detect at least two markers, the multi-assay reagent strip comprises a liquid sealing region, and the liquid sealing region comprises a common liquid region and a dedicated liquid region that are distributed in a length direction of the multi-assay reagent strip;
a multi-channel multi-assay parallel pre-processing apparatus disposed on the mounting frame, wherein the multi-channel multi-assay parallel pre-processing apparatus comprises at least two rows of magnetic rods arranged side by side in a movement direction of the reaction chamber assembly, each row of magnetic rods comprises at least two magnetic rods, and the at least two rows of magnetic rods are configured to transfer magnetic particles in adjacent well positions of the multi-assay reagent strip in the liquid sealing region;
a film breaking assembly disposed on the mounting frame and located at a rear end of the multi-channel multi-assay parallel pre-processing apparatus, wherein the film breaking assembly is configured to break a sealing film of the common liquid region; and
a PMT assembly disposed on the mounting frame and located at a rear end of the film breaking assembly, wherein the reaction chamber assembly is configured to move the multi-assay reagent strip to the PMT assembly, and the PMT assembly is configured to detect a luminescence value of a detection position of the multi-assay reagent strip.

2. The multi-channel multi-assay POCT fully automatic chemiluminescence device according to claim 1, wherein the multi-channel multi-assay parallel pre-processing apparatus comprises:
a gantry disposed on a bottom plate of the mounting frame, wherein the top of the gantry is provided with a first driving element;
a specimen extraction mechanism disposed on the gantry, wherein the specimen extraction mechanism is in transmission connection to the first driving element and may ascend and descend under the drive of the first driving element to aspirate and dispense specimens on the multi-assay reagent strips; and
a specimen pre-processing mechanism disposed on the gantry, wherein the specimen pre-processing mechanism is in transmission connection to the first driving element and may ascend and descend under the drive of the first driving element, and the at least two rows of magnetic rods are disposed on the specimen pre-processing mechanism.

3. The multi-channel multi-assay POCT fully automatic chemiluminescence device according to claim 2, wherein the specimen pre-processing mechanism comprises:
a first mounting frame disposed on the gantry and being in transmission connection to the first driving element, wherein the first driving element may drive the first mounting frame to ascend and descend;
a second driving element disposed on the first mounting frame; and
a pre-processing assembly disposed on the first mounting frame and being in transmission connection to the second driving element, wherein the at least two rows of magnetic rods are disposed on the pre-processing assembly and are in transmission connection to the second driving element, and the second driving element may drive the at least two rows of magnetic rods to ascend and descend to transfer the magnetic particles.

4. The multi-channel multi-assay POCT fully automatic chemiluminescence device according to claim 3, wherein the pre-processing assembly comprises:
a magnetic rod device disposed on the first mounting frame, wherein a plurality of movable through holes are formed in the magnetic rod device, a plurality of first loading heads for loading disposable magnetic separation sleeves are disposed at the bottom of the magnetic rod device, and the plurality of first loading heads communicate with the plurality of movable through holes in a one-to-one manner;
a first push plate disposed above the magnetic rod device, wherein the first push plate is in transmission connection to the second driving element; and
a plurality of connecting rods disposed on the first push plate, wherein the at least two rows of magnetic rods are disposed at free ends of the plurality of connecting rods in a one-to-one manner, and the second driving element may drive the first push plate to drive the magnetic rods to ascend and descend in the corresponding movable through holes, so as to be spaced apart from or pass through the first loading heads.

5. The multi-channel multi-assay POCT fully automatic chemiluminescence device according to claim 4, wherein the magnetic rods are arranged in a plurality of rows, and the plurality of rows of magnetic rods are evenly spaced in a width direction of the first push plate; and
each row of magnetic rods comprises a plurality of magnetic rods, and the plurality of magnetic rods in each row are evenly spaced in a length direction of the first push plate.

6. The multi-channel multi-assay POCT fully automatic chemiluminescence device according to claim 3, wherein the specimen extraction mechanism comprises:
a second mounting frame disposed on the gantry and being in transmission connection to the first driving element, wherein the first driving element may drive the second mounting frame to ascend and descend;
a third driving element disposed on the second mounting frame; and
an extraction assembly disposed on the second mounting frame and being in transmission connection to the third driving element, wherein the third driving element may drive the extraction assembly to ascend and descend to aspirate and dispense the specimens.

7. The multi-channel multi-assay POCT fully automatic chemiluminescence device according to claim 6, wherein the extraction assembly comprises:
an injection device disposed on the second mounting frame, wherein a plurality of injector cavities are formed in the injection device, a plurality of second loading heads for loading tip heads are disposed at the bottom of the injection device, and the plurality of second loading heads communicate with the plurality of injector cavities in a one-to-one manner;
a second push plate disposed above the injection device, wherein the second push plate is in transmission connection to the third driving element; and
a plurality of piston rods disposed on the second push plate, wherein the piston rods are movably and sealingly connected to the injector cavities, and the third driving element is capable of driving the second push plate to drive the piston rods to ascend and descend in the injector cavities to perform aspirating and dispensing actions.

8. The multi-channel multi-assay POCT fully automatic chemiluminescence device according to claim 7, wherein the specimen extraction mechanism comprises:
push rods that penetrate through the injection device in a liftable manner, wherein top ends of the push rods are spaced apart from the second push plate;
elastic elements sleeving the push rods located in the injection device, wherein the elastic elements are simultaneously stretched or compressed when the push rods ascend or descend relative to the injection device; and
a separation plate disposed at the bottom of the injection device, wherein the second loading heads pass through the separation plate, bottom ends of the push rods are connected to the separation plate, and the push rods may drive the separation plate to ascend and descend relative to the second loading heads.

9. The multi-channel multi-assay POCT fully automatic chemiluminescence device according to claim 7, wherein the plurality of injector cavities are evenly spaced in a length direction of the injection device, the plurality of second loading heads are evenly spaced in the length direction of the injection device, and the plurality of piston rods are evenly spaced in a length direction of the second push plate.

10. The multi-channel multi-assay POCT fully automatic chemiluminescence device according to claim 6, wherein the specimen extraction mechanism is opposite to the specimen pre-processing mechanism, the specimen extraction mechanism is located in front of the specimen pre-processing mechanism, and the first driving element, the second driving element, and the third driving element are linearly arranged on the top of the gantry.

11. The multi-channel multi-assay POCT fully automatic chemiluminescence device according to claim 6, wherein the gantry is provided with a movable space, a transmission shaft of the first driving element passes through the movable space, a transmission plate sleeves the transmission shaft, and when the transmission shaft acts, the transmission plate is capable of ascending and descending in the movable space; and
a first connecting portion is disposed on one side of the first mounting frame towards the gantry, a second connecting portion is disposed on one side of the second mounting frame towards the gantry, and both the first connecting portion and the second connecting portion are connected to the transmission plate.

12. The multi-channel multi-assay POCT fully automatic chemiluminescence device according to claim 6, wherein a first slide seat is disposed on one side of the first mounting frame towards the gantry, a first slide rail is disposed on one side of the gantry towards the first mounting frame, the first slide rail extends in a height direction of the gantry, and first slide seat is slidably connected to the first slide rail; and
a second slide seat is disposed on one side of the second mounting frame towards the gantry, a second slide rail is disposed on one side of the gantry towards the second mounting frame, the second slide rail extends in the height direction of the gantry, and the second slide seat is slidably connected to the second slide rail.

13. The multi-channel multi-assay POCT fully automatic chemiluminescence device according to claim 6, wherein a first sensing member is disposed on one side of the top of the first mounting frame, a first detector is disposed on one side of the top of the gantry, and the first sensing member is capable of generating induction or disconnecting induction with the first detector as the first mounting frame ascends and descends; and
a second sensing member is disposed on one side of the top of the second mounting frame, a second detector is disposed on one side of the top of the gantry, and the second sensing member may generate induction or disconnect induction with the second detector as the second mounting frame ascends and descends.

14. The multi-channel multi-assay POCT fully automatic chemiluminescence device according to claim 1, wherein the reaction chamber assembly comprises:
a guide rail disposed on the bottom plate of the mounting frame;
a reaction chamber body disposed on the guide rail, wherein a guide block that is slidably connected to the guide rail is disposed at the bottom of the reaction chamber body, a plurality of accommodation grooves evenly spaced and configured to load the multi-assay reagent strips are disposed on the reaction chamber body, and the accommodation grooves extend in a length direction of the reaction chamber body;
a fourth driving element disposed on the bottom plate of the mounting frame and located at a rear end of the guide rail, wherein a first driving pulley is disposed on a transmission shaft of the fourth driving element;
a mounting seat disposed on the bottom plate of the mounting frame and located at a front end of the guide rail, wherein a first driven pulley is disposed on the mounting seat, and the first driving pulley is in transmission connection to the first driven pulley through a belt; and
a first synchronous belt pressing plate disposed on one side of the reaction chamber body, wherein the first synchronous belt pressing plate is connected to the belt.

15. The multi-channel multi-assay POCT fully automatic chemiluminescence device according to claim 1, wherein the PMT assembly comprises:
a mounting bracket disposed on the bottom plate of the mounting frame;
a third mounting frame disposed on the mounting bracket;
a fourth mounting frame disposed at the bottom of the third mounting frame;
a PMT module disposed on the fourth mounting frame;
a fifth driving element disposed on the top of the third mounting frame and being in transmission connection to the fourth mounting frame, wherein the fifth driving element may drive the fourth mounting frame to ascend and descend so as to drive the PMT module to ascend and descend;
a sixth driving element and a second driven pulley respectively disposed on two sides of the top of the mounting bracket, wherein a transmission shaft of the sixth driving element is provided with a second driving pulley, and the second driving pulley is in transmission connection to the second driven pulley through a belt; and
a second synchronous belt pressing plate disposed on the third mounting frame, wherein the second synchronous belt pressing plate is connected to the belt.

16. The multi-channel multi-assay POCT fully automatic chemiluminescence device according to claim 15, wherein the film breaking assembly comprises:
a seventh driving element disposed on the top of the mounting bracket;
a fifth mounting frame disposed on the mounting bracket and located below the seventh driving element, wherein the fifth mounting frame is in transmission connection to the seventh driving element and can ascend and descend under the drive of the seventh driving element; and
a plurality of film breaking heads disposed at the bottom of the fifth mounting frame, wherein the plurality of film breaking heads are evenly spaced in a length direction of the fifth mounting frame.

17. The multi-channel multi-assay POCT fully automatic chemiluminescence device according to claim 1, wherein the multi-assay reagent strip comprises:
a reagent strip body, wherein the liquid sealing region is disposed on the reagent strip body;
a handle disposed at one end of the reagent strip body; and
a specimen well, a tip head, at least two disposable magnetic separation sleeves, the dedicated liquid region, and the common liquid region, which are disposed on the reagent strip body and sequentially arranged from the handle to the other end of the reagent strip body, wherein
the dedicated liquid region comprises at least two carbon particle-labeled ligands and at least two enzyme-labeled ligands, and the common liquid region comprises a cleaning solution and a luminescent substrate solution.
